# EUROPÄISCHE PATENTSCHRIFT

(11) **EP 3 135 228 B1**
(45) Veröffentlichungstag und Bekanntmachung des Hinweises auf die Patenterteilung: **04.12.2019**
(21) Anmeldenummer: 16001874.3
(22) Anmeldetag: 26.08.2016
(51) Int. Cl.: A61B 17/29

(54) **ARBEITSEINSATZ FÜR EIN MIKROCHIRURGISCHES INSTRUMENT, MIKROCHIRURGISCHES INSTRUMENT UND KRAFT- UND DREHMONENT-ÜBERTRAGUNGSSCHLAUCH**
WORKING UNIT FOR A MICROSURGICAL INSTRUMENT, MICROSURGICAL INSTRUMENT AND FORCE AND TORQUE TRANSMISSION SLEEVE
UNITÉ DE TRAVAIL POUR UN INSTRUMENT DE MICROCHIRURGIE, INSTRUMENT DE MICROCHIRURGIE ET TUYAU DE TRANSFERT A MOMENT DE FORCE ET DE COUPLE

(30) Priorität: 26.08.2015 DE 102015010970
(43) Veröffentlichungstag der Anmeldung: 01.03.2017
(73) Patentinhaber: Karl Storz SE & Co. KG, 78532 Tuttlingen (DE)
(72) Erfinder: Jochen, Stefan, 88639 Wald (DE); Volkmer, Dominik, 78567 Fridingen (DE)
(74) Vertreter: mepat Patentanwälte

(56) Entgegenhaltungen:
- EP-A1- 2 837 340
- EP-A2- 2 767 242
- WO-A1-95/18574
- WO-A2-2008/045333
- US-A1- 2011 071 347
- US-A1- 2013 267 936
- US-A1- 2014 005 662

## Beschreibung

Die nachfolgende Erfindung bezieht sich auf einen Arbeitseinsatz für ein mikrochirurgisches Instrument, auf das mikrochirurgische Instrument selbst und auf einen Kraft- und Drehmomentübertragungsschlauch für den Arbeitseinsatz.

Minimalinvasive Operationsmethoden ersetzen in zunehmendem Maße die klassische offene Operationstechnik, wobei neben einem besseren kosmetischen Ergebnis (kürzere Hautnaht) vor allem die geringere Schädigung umliegenden Gewebes und damit verkürzte Verweildauern im Krankenhaus vorteilhaft sind. Neben rein mechanischen Instrumenten mit maulförmigen Arbeitseinsätzen, etwa Scheren, Zangen, Nadelhaltern etc., sind auch elektrochirurgische Instrumente für die Mikrochirurgie bekannt. Hierbei wird zwischen dem zu schneidenden und/oder zu haltenden Gewebe eine hochfrequente Spannung (HF-Spannung) angelegt, die durch Erwärmung des umliegenden Gewebes zu einer Blutungsstillung (Koagulation) führt.

Ein elektrochirurgisches Instrument wird beispielsweise in DE 602 26 015 T2 offenbart.

Die Werkzeugenden solcher elektrochirurgischer Instrumente sollen neben der Koagulationsfunktion sämtliche Funktionen von klassischen mikrochirurgischen Instrumenten aufweisen, so etwa Schwenkbarkeit und/oder Abwinkelbarkeit sowie gute Kraftrückmeldung aus dem Branchenbereich.

Der zur Verfügung stehende Bauraum im Inneren des Schafts ist bei den gängigen Schaftdurchmessern von wenigen Millimetern eng. Man ist daher dazu übergegangen, ein kombiniertes Kraft- und Drehmomentübertragungselement einzusetzen, über das sowohl die Offen- und Schließbewegung der Branchen als auch eine Drehbewegung des Arbeitseinsatzes gesteuert werden kann. Es ist hierbei üblich, neben starren Rohren in abwinkelbaren Schaftbereichen einen torsionssteifen Kraft- und Drehmomentübertragungsschlauch einzusetzen. Ein mikrochirurgisches Instrument, das als Kraftübertragungselement einen Schlauch einsetzt, wird in EP 2837340 A1 offenbart.

Bei solchen mikrochirurgischen Instrumenten werden die maximal an den Branchen erreichbaren Betätigungskräfte jedoch durch elastische Verformungen des Kraft- und Drehmomentobertragungsschlauchs begrenzt, was für den Operateur, insbesondere beim Fassen dickerer Gewebeteile, unbefriedigend ist. Ferner ist bei elektrochirurgischen Instrumenten, die nach diesem Prinzip aufgebaut sind, die Regulierung der zur Koagulation eingesetzten Stromdichle nicht genau möglich, denn die Regulierung der zur Koagulation eingesetzten Stromdichte erfolgt im Wesentlichen über den auf das gefasst Gewebe ausgeübten Druck (bei stärkerem Druck tritt ein kleinerer Übergangswiderstand auf und umgekehrt). Um die Stromdichte möglichst genau dosieren zu können, ist daher eine gute Kraftrückkopplung notwendig, die durch die elastische Verformungen, d. h., eine zu geringe Steifigkeit des Schlauchs, gehindert wird.

Ausgehend von diesem Stand der Technik liegt der vorliegenden Erfindung die Aufgabe zu Grunde, einen verbesserten Arbeitseinsatz für ein mikrochirurgisches Instrument zu schaffen, der insbesondere höhere Betätigungskräfte an den Branchen ermöglicht und eine verbesserte Kraftrückkopplung erlaubt und, bei Ausbildung als elektrochirurgisches Instrument, eine verbesserte Regulierbarkeit der Stromdichte.

Diese Aufgabe wird durch einen Arbeitseinsatz für ein mikrochirurgisches Instrument mit den Merkmalen des unabhängigen Anspruchs 3 gelöst.

Darüber hinaus ergibt sich die Aufgabe, ein mikrochirurgisches Instrument zu schaffen, mit dem erhöhte Betätigungskräfte erreichbar sind, das eine gute Kraftrücckopplung aus dem Operationsgebiet erlaubt und leicht bedienbar ist.

Diese Aufgabe wird durch ein mikrochirurgisches Instrument mit den Merkmalen des Anspruchs 8 gelöst.

Schließlich ergibt sich noch die Aufgabe, einen Kraft- und Drehmomenlübertragungsschlauch zu schaffen, mit dem bestehende Arbeitseinsätze nachgerüstet werden können, um diese in o. g. Weise zu verbessern.

Diese Aufgabe wird durch einen Kraft- und Drehrnomentübertragungsschlauch mit den Merkmalen des Anspruchs 1 gelöst.

Bevorzugte Weiterbildungen werden jeweils durch die Unteransprüche beschrieben.

Der erfindungsgemäße Arbeitseinsatz für ein mikrochirurgisches Instrument weist in einer ersten Ausführungsform einen proximalen Kopplungsabschnitt auf, der mit einer Handhabe koppelbar ist. Von dem Kopplungsabschnitt erstreckt sich ein hohler Schaft zu einem distalen Werkzeugende, an dem eine um die Längsachse des Schafts drehbar gelagerte maulförmige Arbeitsvorrichtung mit zumindest zwei gegeneinander verschwenkbaren Branchen angeordnet ist. Im Inneren des Schafts verläuft eine Kraft- und Drehmomentübertragungsvorrichtung von dem Kopplungsabschnitt zu dem distalen Werkzeugende, der zur Übertragung einer Drehbewegung und von Offen- und Schließkräften operativ mit der Arbeitsvorrichtung verbunden ist. Die Kraft- und Drehmomentübertragungsvorrichtung ist zumindest entlang eines Längenabschnitts als Kraft- und Drehmomentüberlragungsschlauch ausgebildet, der in wenigstens einem Längenabschnitt mit zumindest einem in Längsrichtung des Kraft- und Drehmomentübertragungsschlauches verlaufenden Zugelement versteift ist.

Mit "zumindest ein Abschnitt" kann dabei ein zusammenhängender Abschnitt oder aber es können zwei oder mehr getrennte Abschnitte gemeint sein. Der Teil der Kraft- und Drehmomentübertragungsvorrichtung, der als Kraft- und Drehmomentübertragungsschlauch ausgebildet ist, kann beispielsweise in einem abwinkelbaren Abschnitt des Schafts liegen, da der Schlauch flexibel ist und der Abwinkelung folgt. Es ist aber nicht ausgeschlossen, dass die Kraft- und Drehmomentübertragungsvorrichtung auf ihrer Gesamtlänge aus dem Kraft- und Drehmomentübertragungsschlauch besteht. Bei dem Teil der Kraft- und Drehmomentübertragungsvorrichtung, der nicht als Schlauch ausgebildet ist, kann es sich beispielsweise um ein starres Rohr handeln.

Unter "Arbeitseinsatz", also Einsatz zum Arbeiten, wird hierin der Teil eines mikrochirurgischen Instruments verstanden, der den Schaft und die maulförmige Arbeitsvorrichtung am distalen Ende (Schere, Greifbacken, Nadelhalter o. ä.) aufweist. Der Arbeitseinsatz im Sinne der vorliegenden Erfindung umfasst zudem ein oder mehrere Kraft- bzw. Drehmomentübertragungsmittel die in dem Schaft verlaufen und mit denen eine Betätigungskraft von einem Betätigungselement der Handhabe auf die distale Arbeitsvorrichtung ausgeübt werden kann. Der Arbeitseinsatz wird, um ein einsatzfähiges mikrochirurgisches Instrument zu erhalten, mit der Handhabe gekoppelt.

Die Arbeitsvorrichtung kann beispielsweise in einem Winkelbereich zwischen 0 und 90 °, 0 bis 180 ° oder in einem noch größeren Winkelbereich, sogar unbegrenzt, um die Längsachse des Schafts gedreht werden.

Der Kraft- und Drehmomentübertragungsschlauch soll möglichst torsionssteif ausgeführt werden, sodass Drehbefehle von der Handhabe möglichst direkt und ohne Verfälschungen zu der Arbeitsvorrichtung übertragen werden können. Es ist dem Fachmann bekannt, wie ein Kraft- und Drehmomentübertragungsschlauch zu bauen ist, der diese Eigenschaften hat (vgl. Prinzip einer biegsamen Welle oder eines Brauseschlauchs).

Der Schaft des erfindungsgemäßen Arbeitseinsatzes hat beispielsweise einen Durchmesser von einigen wenigen Millimetern, typischerweise etwa 2,5 bis 8 mm; es sind aber auch größere oder kleiner Bauformen denkbar.

Indem erfindungsgemäß eine Zugverstärkung des Kraft- und Drehmomentübertragungsschlauchs vorgesehen ist, wird dessen Zugsteifigkeit deutlich erhöht, was zu geringeren Verformungen führt und die auf die Branchen der Arbeitsvorrichtung ausübbaren Kräfte erhöht sowie die Kraftrückkopplung verbessert. Mit einem erfindungsgemäßen Arbeitseinsatz eines bestimmten Durchmessers ist es daher möglich, deutlich dickere Gewebe zu schneiden oder zu halten bzw. höhere Nahtkräfte auszuüben als mit einem bekannten Arbeitseinsatz der gleichen Durchmesserklasse. Vorteilhaft betreffen die konstruktiven Änderungen erfindungsgemäß nur den Kraft- und Drehmomentübertragungsschlauch an sich, während die weiteren Bestandteile des Arbeitseinsatzes unverändert bleiben; somit kann durch eine überraschend kleine und daher kostengünstig umsetzbare konstruktive Anpassung eine vergleichsweise große Nutzwertsteigerung erreicht werden.

Die Branchen der Arbeitsvorrichtung können in ihren distalen Endbereichen jeweils einen Wirkabschnitt aufweisen, bevorzugt eine Schneide oder eine Greiffläche; bei dem Arbeitseinsatz kann es sich daher, je nach Ausbildung der Wirkabschnitte, beispielsweise um einen Nadelhalter, eine Fasszange, eine Schere, eine Biopsiezange, eine Spreizzange oder eine Präparierzange handeln.

Es ist hierbei möglich, dass eine der Branchen feststehend und die andere Branche beweglich ist, oder dass beide Branchen beweglich sind und schwenkbar in einer separaten Basis der Arbeitsvorrichtung gelagert sind. Es kann dabei insbesondere vorgesehen sein, dass der Kraft- und Drehmomentübertragungsschlauch zur Einleitung der Drehbewegung mit der Basis verbunden ist.

In einer weiteren Ausführungsform kann das Zugelement entweder innerhalb des Kraft- und Drehmomentübertragungsschlauches, etwa am Innendurchmesser, oder außerhalb des Kraft- und Drehmomentübertragungsschlauches, etwa am Außendurchmesser, angeordnet sein. Ferner kann das Zugelement zumindest in einem proximalen und einem distalen Endbereich des Kraft- und Drehmomentübertragungsschlauches mit dem Kraft- und Drehmomentübertragungsschlauch verbunden sein, bevorzugt verschweißt. Bei dem Kraft- und Drehmomentübertragungsschlauch kann es sich beispielsweise um ein Seil, etwa ein Metallseil, einen Draht, eine Faser oder ein Faserbündel handeln.

Wenn das Zugelement in dem Kraft- und Drehmomentübertragungsschlauch angeordnet wird, werden vorteilhaft die Außenabmessungen sowie die Anschlussbereiche beibehalten und der Einsatz des versteiften Kraft- und Drehmomentübertragungsschlauches erfordert keine Umkonstruktionsmaßnahmen an dem Arbeitseinsatz. Indem sich das Zugelement über die komplette Länge des Kraft- und Drehmomentübertragungsschlauches erstreckt und nur in den jeweiligen Endbereichen verbunden ist, wird der Kraft- und Drehmomentübertragungsschlauch auf dem wesentlichen Teil seiner Länge von der Übertragung von Zugkräften freigestellt, woraus deutlich geringere Dehnungen resultieren, sodass die Kraftrückkopplungseigenschaften verbessert und die maximale Fasskraft erhöht werden.

In einer noch weiteren Ausführungsform kann die Arbeitsvorrichtung über ein Gelenk mit dem Schaft verbunden sein, Über das die Arbeitsvorrichtung um eine normal zur Längsachse des Schafts verlaufende Drehachse abwinkelbar ist. Alternativ oder zusätzlich kann der Schaft in einem abwinkelbaren Schaftabschnitt zumindest ein Gelenk aufweisen, insbesondere zwei oder mehr Gelenke, über die dieser um eine normal zur Längsachse des Schafts verlaufende Drehachse abwinkelbar ist. In dem abwinkelbaren Schaftabschnitt ist die Kraft- und Drehmomentübertragungsvorrichtung vorteilhafter Weise als Kraft- und Drehmomentübertragungsschlauch ausgebildet, um der Abknickbewegung folgen zu können. Der Kraft- und DrehmomentÜbertragungsschlauch kann insbesondere durch die Gelenke hindurch geführt sein. Ferner kann der Schaft in einem oder mehreren Längenabschnitt(en) biegsam ausgeführt sein.

Darüber hinaus kann es sich bei dem Arbeitseinsatz um einen elektrochirurgischen Arbeitseinsatz handeln, wobei sich entlang des Schafts eine oder mehrere elektrische Verbindungsleitung(en) von dem Kopplungsabschnitt zu dem distalen Werkzeugende erstrecken, die mit einer oder beiden Branchen elektrisch verbunden sind. "Elektrisch verbunden" schließt eine direkte Verbindung mit den Branchen als auch eine mittelbare Verbindung über dazwischengeschaltete Übertragungselemente mit ein. Ob eine oder zwei elektrische Verbindungsleitungen vorgesehen sind, richtet sich danach, ob das Instrument als Unipolar- oder als Bipolarinstrument ausgeführt ist. Wenn es sich um ein Bipolarinstrument handelt, ist je eine Verbindungsleitung mit einer Branche verbunden, während beim Unipolarinstrument nur eine der Branchen mit der elektrischen Verbindungsleitung kontaktiert wird, während die andere mit der Neutralelektrode verbunden ist, die wiederum bei der Anwendung möglichst großflächig mit dem Patientenkörper verbunden wird. Der Fachmann weiß über die einzusetzenden Verbindungsleitungen Bescheid; er wird diese in Abhängigkeit von Stromstärke, Spannung und der auftretenden Frequenzen der HF-Spannung auswählen.

Es kann vorgesehen sein, dass die elektrische Verbindungsleitung bzw. die zwei Verbindungsleitungen im Inneren des Kraft- und Drehmomentübertragungsschlauchs verlaufen. Insbesondere kann vorgesehen sein, dass die Verbindungsleitung(en) an einer dem Zugelement gegenüberliegenden Umfangsposition des Kraft- und Drehmomentübertragungsschlauchs angeordnet sind oder aber zentrisch verlaufen. Es ist vor Allem darauf zu achten, einen ausreichenden Abstand zwischen dem Zugelement und der elektrischen Verbindungsleitung vorzusehen, sodass aufgrund von Relativbewegungen die elektrische Verbindungsleitung bzw. die Ummantelung/Isolierung nicht beschädigt wird.

Das erfindungsgemäße mikrochirurgische Instrument weist in einer ersten Ausführungsform eine proximale Handhabe mit zumindest einem Betätigungselement und einen distalen erfindungsgemäßen Arbeitseinsatz auf, der mit seinem Kopplungsabschnitt operativ mit einer korrespondierenden Kopplungsvorrichtung der Handhabe gekoppelt ist. Der Arbeitseinsatz kann dabei wahlweise dauerhaft oder auswechselbar mit der Handhabe verbunden sein.

Bei der Handhabe kann es sich beispielsweise um einen Pistolengriff oder Pinzettengriff handeln, an der zwei mechanische Betätigungselemente angeordnet sind: Ein erstes Betätigungselement zur Ausübung eines Drehmoments auf die Kraft- und Drehmomentübertragungsvorrichtung des Arbeitseinsatzes und ein zweites Betätigungselement zur Ausübung einer Kraft in Längsrichtung der Kraft- und Drehmomentübertragungsvorrichtung des Arbeitseinsatzes. Die Handhabe kann beispielsweise auch eine Rückstellvorrichtung zur Rückstellung der Betätigungsvorrichtung(en) in eine Ruhestellung haben. Bei einem oder beiden der mechanischen Betätigungselemente kann es sich um ein manuelles Betätigungselement handeln, etwa ein Drehrad, ein Hebel, ein Druckknopf. Alternativ oder zusätzlich kann eines oder beide der mechanischen Betätigungselemente, ein Betätigungselement sein, über das eine Antriebsvorrichtung aktivierbar ist, die dazu ausgebildet ist, auf den Kraft- und Drehmomentübertragungsschlauch des Arbeitseinsatzes einzuwirken. Bei der Antriebsvorrichtung kann es sich bspw. um einen Dreh- und/oder Linearantrieb handeln, etwa einen elektrischen oder pneumatischen Dreh- und/oder Linearantrieb.

Wenn der Drehantrieb der Handhabe zur Erzeugung des Drehmoments für die Drehbewegung der Arbeitsvorrichtung um die Längsachse (=Rollen) des Schafts eingesetzt wird, kann vorteilhafter Weise die Arbeitsvorrichtung unbegrenzt um die Längsachse des Schafts rotiert werden, ohne diese wieder zurückdrehen zu müssen, was bei manueller Betätigung nicht ohne weiteres möglich ist.

Bei Ausbildung des mikrochirurgischen Instruments als elektrochirurgisches Instrument kann die Handhabe mit einer Spannungsquelle, etwa einer HF-Spannungsquelle, verbunden sein. Die Handhabe hat dann zweckmäßiger Weise eine elektrische Kopplungsvorrichtung, die mit einem elektrischen Anschluss des Arbeitseinsatzes verbunden ist bzw. verbindbar ist. Es ist ferner auch möglich, dass an der Handhabe ein elektrisches Betätigungselement vorliegt, um einen Stromfluss von der Spannungsquelle zu dem Arbeitseinsatz zu aktivieren und/oder zu deaktivieren.

Der erfindungsgemäße Kraft- und Drehmomentübertragungsschlauch für einen Arbeitseinsatz eines mikrochirurgischen Instruments ist gemäß einer ersten Ausführungsform in wenigstens einem zwischen den beiden Enden liegenden Abschnitt mit zumindest einem in Längsrichtung des Kraft- und Drehmomentübertragungsschlauches verlaufenden Zugelement versteift. Hierdurch wird die Steifigkeit in Zugrichtung erhöht, sodass ein Arbeitseinsatz respektive mikrochirurgisches Instrument, in dem der Kraft- und Drehmomenlübertragungsschlauch eingesetzt wird, höhere Arbeitskräfte ermöglicht und dabei auf Grund von verbesserten Kraftrückkopplungseigenschaften komfortabel zu bedienen ist. Das Zugelement kann dabei über die komplette Länge des Kraft- und Drehmomentübertragungsschlauchs verlaufen oder nur in einem Teilabschnitt.

In einer weiteren Ausführungsform des Kraft- und Drehmomentübertragungsschlauchs kann das Zugelement innerhalb des Kraft- und Drehmomentübertragungsschlauchs, etwa am Innendurchmesser, oder außerhalb des Kraft- und Drehmomentübertragungsschlauchs, etwa am Außendurchmesser, angeordnet sein. Bei Anordnung in dem Kraft- und Drehmomentübertragungsschlauch bleiben vorteilhaft die äußeren Abmessungen und die Anschlussbereiche zur Krafteinleitung und -ausleitung erhalten, sodass der erfindungsgemäße Kraft- und Drehmomentübertragungsschlauch eins zu eins als Ersatz in Bestandsinstrumenten eingesetzt werden kann.

Alternativ oder zusätzlich kann das Zugelement in einem proximalen und distalen Endbereich des Kraft- und Drehmomentübertragungsschlauches mit dem Kraft- und Drehmomentübartragungsschlauch verbunden sein, beispielsweise direkt verschweißt. Es ist aber nicht ausgeschlossen, dass das Zugelement an weiteren Orten, etwa auch auf seiner kompletten Länge, mit dem Kraft- und Drehmomentübertragungsschlauch verbunden ist. Bei dem Zugelement kann es sich um ein Seil, etwa ein Metailseil, einen Draht, eine Faser oder ein Faserbündel handeln. Vorgenannte Aufzählung ist allerdings nur als beispielhaft zu verstehen; es ist selbstverständlich möglich, andere Zugelemente einzusetzen, mit denen der gleiche Zweck einer Zugversteifung des Kraft- und Drehmomentübertragungsschlauchs erreicht werden kann.

Diese und weitere Vorteile werden durch die nachfolgende Beschreibung unter Bezug auf die begleitenden Figuren dargelegt. Der Bezug auf die Figuren in der Beschreibung dient der Unterstützung der Beschreibung und dem erleichterten Verständnis des Gegenstands. Gegenstände oder Teile von Gegenständen, die im Wesentlichen gleich oder ähnlich sind, können mit denselben Bezugszeichen versehen sein. Die Figuren sind lediglich schematische Darstellungen von Ausführungsbeispielen der Erfindung.

Es zeigt:
- **Fig. 1**: eine perspektivische Ansicht des mikrochirurgischen Instruments,
- **Fig. 2**: eine perspektivische Detailansicht des mikrochirurgischen Instruments,
- **Fig. 3**: einen Längsschnitt des distalen Endbereichs des Schafts,
- **Fig. 4**: einen anderen Längsschnitt des distalen Endbereichs des Schafts,
- **Fig. 5**: einen Querschnitt des Schafts in dem abwinkelbaren Schaftabschnitt.
- **Fig. 6**: eine Detailansicht des Querschnitts des Schafts in dem abwinkelbaren Schaftabschnitt.

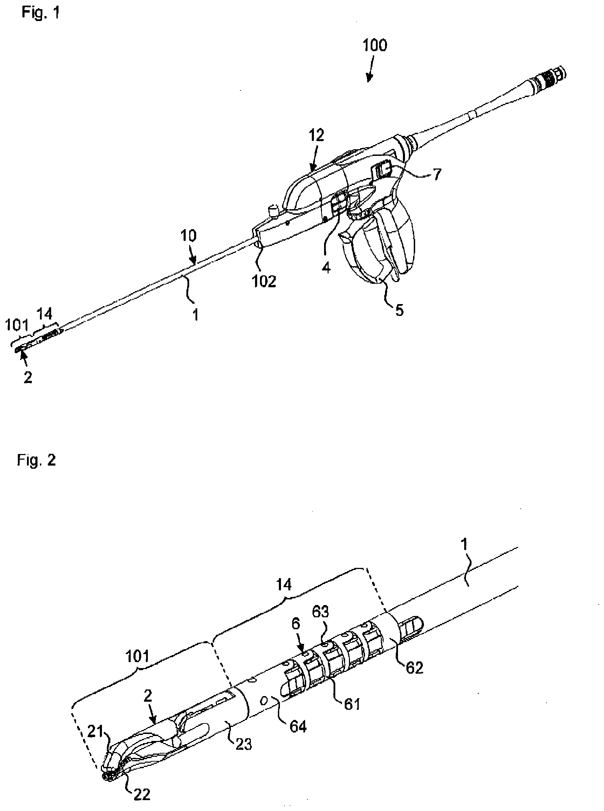

Die Erfindung betrifft einen Arbeitseinsatz 10 für ein mikrochirurgisches Instrument 100, wie es in den **Fig. 1** und **Fig. 2** perspektivisch gezeigt ist. Der Arbeitseinsatz 10 besteht hier aus Arbeitsvorrichtung 2 und verbindendem Schaft 1. Das mikrochirurgische Instrument 100 besteht im Wesentlichen aus einer Handhabe 12 und dem angekoppelten Arbeitseinsatz 10.

Über die Handhabe 12, bzw. genauer, deren Betätigungselemente 4,5,7, lassen sich die Funktionen des Arbeitseinsatzes 10 steuern. Vorliegend handelt es sich bei den Funktionen um: Drehen der Arbeitsvorrichtung 2 um die Längsachse des Schafts 1, Öffnen/Schließen der Branchen 21,22 (siehe **Fig. 2**) der Arbeitsvorrichtung 2 und Abwinkeln des abwinkelbaren Schaftabschnitts 14. Die Handhabe 12 hat drei Betätigungselemente 4,5,7 (**Fig. 1**), wobei über das erste (manuelle) Betätigungselement 4 die Arbeitsvorrichtung 2 um die Längsachse rotiert werden kann, über das zweite (manuelle) Betätigungselement 5 die Branchen 21,22 geöffnet und geschlossen werden können und über das Betätigungselement 7, das ein elektrisches Betätigungselement 7 ist, ein Antrieb aktiviert werden kann, über den der Schaft 1 in dem abwinkelbaren Schaftabschnitt 14 abgewinkelt werden kann.

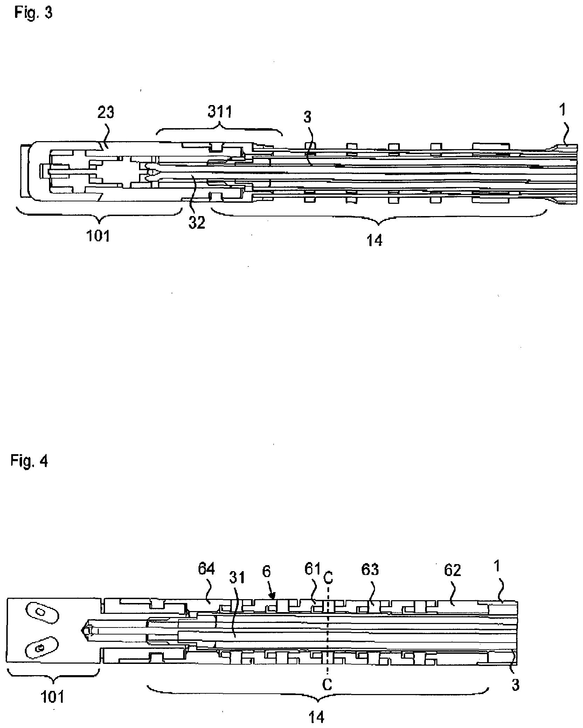

An einem distalen Ende des Arbeitseinsatzes 10 ist das Werkzeugende 101, in dem die Arbeitsvorrichtung 2 mit dem Schaft 1 gekoppelt ist, der der mechanischen Verbindung mit der Handhabe 12 dient. Der Schaft 1 ist wiederum an seinem proximalen Kopplungsabschnitt 102 mit der Handhabe 12 gekoppelt. Der Schaft 1 ist hohl und beispielsweise als Rohr aus einem Metall oder Kunststoff ausgebildet. Darin ist eine Kraft- und Drehmomentübertragungsvorrichtung geführt, die zur Kraft- und Drehmomentübertragung von der Handhabe 12 zur Arbeitsvorrichtung 2 dient. Bei der Kraft- und Drehmomentübertragungsvorrichtung handelt es sich beispielsweise um ein Rohr oder einen Stab. In dem abwinkelbaren Schaftabschnitt 14 ist die Kraft- und Drehmomentübertragungsvorrichtung jedoch als Kraft- und Drehmomentübertragungsschlauch 3 (siehe **Fig. 4**) ausgebildet, der biegsam ist, um der Abwinkelung folgen zu können.

Der abwinkelbare Schaftabschnitt 14 ist in **Fig. 2** im Detail dargestellt. Die Abwinklung wird über mehrere Gelenke 6 erreicht, die aus den Gliedern 61,62,64 bestehen. Die Glieder 61,62,64 sind unter einander schwenkbar über eine Achse 63, hier einen Nietschaft 63, verbunden. Am proximalen Ende (in der Abbildung rechts) des abwinkelbaren Schaftabschnitts 14 ist ein proximales Anschschlussglied 62 angeordnet und an dem distalen Ende des abwinkelbaren Schaftabschnitts 14 ein distales Anschlussglied 64, wobei die Anschlussglieder 62,64 jeweils die Verbindung zu dem Schaft 1 oder der Arbeitsvorrichtung 2 herstellen. Zwischen dem proximalen Anschlussglied 62 und dem distalen 62 Anschlussglied befinden sich Verbindungsglieder 61, hier vier Stück. In einer nicht gezeigten alternativen Ausführungsform können es selbstverständlich auch mehr oder weniger Verbindungsglieder 61 sein, je nach vorzusehendem Knickwinkel und -radius.

Die Arbeitsvorrichlung 2 hat zwei öffen- und schließbare Branchen 21,22 und ist hier als Fasszange ausgebildet, kann in anderen Ausführungsformen aber beispielsweise auch ein Nadelhalter, eine Schere o. ä. sein. Die beiden Branchen 21,22 sind in einer Basis 23 der Arbeitsvorrichtung 2 schwenkbar gelagert. Die Basis 23 der Arbeilsvorrichtung 2 ist ihrerseits um die Längsachse des Schafts 1 drehbar gegenüber dem Schaft 1 bzw. dem distalen Anschlussglied 64 gelagert.

Bei dem gezeigten Arbeitseinsatz 10 handelt es sich um einen elektrochirurgischen Arbeitseinsatz 10, der es ermöglicht zwischen den beiden Branchen 21,22 einen Stromfluss einzuleiten.

Hierzu verlaufen im Inneren der Kraft- und Drehmomentübertragungsvorrichtung und des Kraft- und Drehmomentübertragungsschlauchs 3 zwei elektrische Verbindungsleitungen 32, die jeweils mit einer der Branchen 21,22 elektrisch leitend verbunden sind, siehe hierzu **Fig. 3****.** Ein (HF)-Stromfluss durch das mit den Branchen 21,22 gefasste Gewebe erfolgt dann zwischen Branchen 21,22. In einer nicht figurativ gezeigten Ausführungsform kann es sich bei dem Arbeitseinsatz 10 auch um einen Arbeitseinsatz 10 eines Unipolarinstruments handeln, d. h., es verläuft in der Kraft- und Drehmomentübertragungsvorrichtung und dem Kraft- und Drehmomentübertragungsschlauch 3 nur ein Kabel, wobei der Stromfluss in diesem Fall zwischen den Branchen 21,22 und einer Neutralelektrode erfolgt, die auf dem Patienten zu befestigen ist.

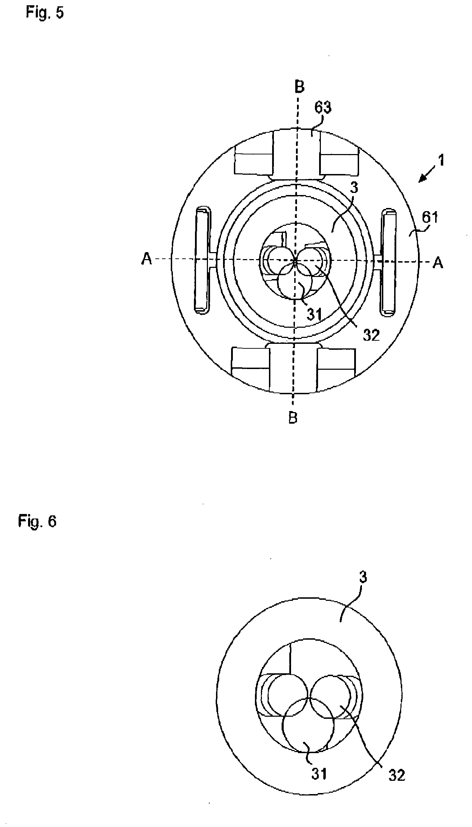

Die **Fig. 3** und **Fig. 4** zeigen zwei Längsschnitte des distalen Endabschnitts des Arbeitseinsatzes 10, wobei die Schnittebenen in **Fig. 5** eingezeichnet sind. **Fig. 3** stellt den Schnitt A-A dar und **Fig. 4** den Schnitt B-B.

In **Fig. 3** sind insbesondere die elektrischen Verbindungsleitungen 32, die sich zu dem distalen Werkzeugende 101 erstrecken, zu erkennen. An ihren distalen (in der Abbildung linken) Enden sind die elektrischen Verbindungsleitungen 32 jeweils elektrisch leitend mit einer der Branchen 21,22 (siehe **Fig. 2**) verbunden. In dem abwinkelbaren Schaftabschnitt 14 ist der Kraft- und Drehmomentübertragungsschlauch 3 zu sehen, in dem die elektrischen Verbindungsleitungen 32 geführt sind. Zur Einleitung der Kräfte und Momente ist der Kraft- und Drehmomentübertragungsschlauch 3 in seinem distalen Ende 311 operativ mit der Arbeitsvorrichtung 2 gekoppelt.

In **Fig. 4****,** deren Schnittebene um 90° zur **Fig. 3** gedreht ist, ist der mechanische Aufbau des abwinkelbaren Schaftabschnitts 14 gut zu erkennen. Die Gliederelemente 61,62,64, die die Gelenke 6 bilden, sind unter einander über die Achsen 63 bzw. Nietschäfte 63 schwenkbar verbunden. Innerhalb der Glieder 61,62,64 ist der Kraft- und Drehmomentübertragungsschlauch 3 geführt.

Der Kraft- und Drehmomentübertragungsschlauch 3 ist zur Versteifung in Zugrichtung (Längsachse) mit einem Zugelement 31 versteift, das in dem distalen Endbereich 311 (siehe **Fig. 3**) und dem proximalen Endbereich (figurativ nicht gezeigt) jeweils mit dem Kraft- und Drehmomentübertragungsschlauch 3 verschweißt ist. Vorteilhafter Weise wird der Kraft- und Drehmomentübertragungsschlauch 3 dadurch auf einem Großteil seiner Länge von Zugkräften freigestellt, was zu einer höheren Zugsteifigkeit führt und damit für den Arbeitseinsatz 10 höhere erreichbare Maulkräfte und eine verbesserte Kraftrückkopplung bedeutet.

In den **Fig. 5** und **Fig. 6** ist schließlich ein Querschnitt des Arbeitseinsatzes 10 gezeigt, der bezüglich der Schnittebene C-C (siehe **Fig. 4**) erzeugt wurde. Bei Fig. 6 handelt es sich um eine Detailansicht, die nur den Kraft- und Drehmomenlübertragungsschlauch 3 zeigt. Die Schnittebene liegt demnach in dem abwinkelbaren Schaftabschnitt 14, in dem der Kraft- und Drehmomentübertragungsschlauch 3 durch die Glieder 61 geführt ist. Hierdurch ist das Innere des Kraft- und Drehmomentübertragungsschlauchs 3 gut zu erkennen, insbesondere die Zugverstärkung 31 und die beiden darin geführten elektrischen Verbindungsleitungen 32.

Die eingezeichneten Überlappungen des Kraft- und Drehmomentübertragungsschlauchs 3 und der elektrischen Verbindungsleitungen 32 sind unbeachtlich und resultieren lediglich aus der Zeichnungsableitung. In der gezeigten Schnittebene C-C überlappen sich die elektrischen Verbindungsleitungen 32 und der Kraft- und Drehmomentübertragungsschlauch 3 nicht, sondern sind jeweils am Innenquerschnitt des Kraft- und Drehmomentübertragungsschlauchs 3 angeordnet.

Der Kraft- und Drehmomentübertragungsschlauch 3 kann beispielsweise aus spiralförmig gewickelten Streifen aufgebaut sein, die an ihren jeweiligen Längsachsen über eine Bördelung verbunden sind, die aufragende Rippen bildet (figurativ nicht gezeigt). Zwischen den durch die Bördelung gebildeten Rippen befinden sich Sicken, in denen jeweils eine umlaufende Drahtwicklung zur Erhöhung der Torsionssteifigkeit und zur Verringerung der Kickgefahr angeordnet sein kann. Der Kraft- und Drehmomentübertragungsschlauch 3 kann aber selbstverständlich auch nach einem anderen Grundprinzip aufgebaut sein, z. B. wie eine biegsame Welle oder ein Duschschlauch, der dann mit dem Zugelement 31 versteift wird.

### BEZUGSZEICHENLISTE

- 100: Mikrochirurgisches Instrument
- 10: Arbeitseinsatz
- 101: Distales Werkzeugende
- 102: Proximaler Kopplungsabschnitt
- 1: Schaft
- 12: Handhabe
- 14: Abwinkelbarer Schaftabschnitt
- 2: Arbeitsvorrichtung
- 21: Branche der Arbeitsvorrichtung
- 22: Branche der Arbeitsvorrichtung
- 23: Basis der Arbeitsvorrichtung
- 3: Kraft- und Drehmomentübertragungsschlauch
- 31: Zugelement
- 311: Distaler Endbereich des Kraft- und Drehmomentübertragungsschlauchs
- 32: Elektr. Verbindungsleitung
- 4: Erstes manuelles Betätigungselement
- 5: Zweites manuelles Betätigungselement
- 6: Gelenk des Schafts
- 61: Verbindungsglied des abwinkelbaren Schaftabschnitts
- B2: Proximales Anschlussglied des abwinkelbaren Schaftabschnitts
- 63: Achse/Nietschaft
- 64: Distales Anschlussglied des abwinkelbaren Schaftabschnitts
- 7: Elektrisches Betätigungselement

## Patentansprüche

1. Kraft- und Drehmomentübertragungsschlauch (3) für einen Arbeitseinsatz (10) für ein mikrochirurgisches Instrument (100),
**dadurch gekennzeichnet, dass**
der Kraft- und Drehmomentübertragungsschlauch (3) zumindest in einem zwischen seinem beiden Enden liegenden Abschnitt mit zumindest einem in Längsrichtung des Kraft- und Drehmomentübertragungsschlauches (3) verlaufenden Zugelement (31) versteift ist.

2. Kraft- und Drehmomentübertragungsschlauch (3) nach Anspruch 1,
**dadurch gekennzeichnet, dass**
das Zugelement (31)
- innerhalb, bevorzugt am Innendurchmesser, oder außerhalb, bevorzugt am Außendurchmesser, des Kraft- und Drehmomentübertragungsschlauches (3) angeordnet ist und/oder
- zumindest in einem proximalen und einem distalen (311) Endbereich des Kraft- und Drehmomentübertragungsschlauches (3) mit dem Kraft- und Drehmomentübertragungsschlauch (3) verbunden ist, bevorzugt verschweißt und/oder
- ein Seil, bevorzugt ein Metallseil, ein Draht, eine Faser oder ein Faserbündel ist.

3. Arbeitseinsatz (10) für ein mikrochirurgisches Instrument (100),
- mit einem proximalen Kopplungsabschnitt (102), der mit einer Handhabe (12) koppelbar ist,
- wobei sich von dem Kopplungsabschnitt (102) ein hohler Schaft (1) zu einem distalen Werkzeugende (101) erstreckt, an dem eine um die Längsachse des Schafts (1) drehbar gelagerte maulförmige Arbeitsvorrichtung (2) mit zumindest zwei gegeneinander verschwenkbaren Branchen (21,22) angeordnet ist,
- und wobei sich in dem Inneren des Schafts (1) von dem Kopplungsabschnitt (102) zu dem distalen Werkzeugende (101) eine Kraft- und Drehmomentübertragungsvorrichtung erstreckt, die zumindest entlang eines Längenabschnitts als Kraft- und Drehmomentübertragungsschlauch (3) ausgebildet ist, wobei die Kraft- und Drehmomentübertragungsvorrichtung zur Übertragung einer Drehbewegung und von Offen- und Schließkräften operativ mit der Arbeitsvorrichtung (2) verbunden ist,
**dadurch gekennzeichnet, dass**
der Kraft- und Drehmomentübertragungsschlauch ein Kraft- und Drehmomentübertragungsschlauch (3) nach Anspruch 1 oder 2, der zumindest in einem Längenabschnitt mit zumindest einem in Längsrichtung verlaufenden Zugelement (31) versteift ist.

4. Arbeitseinsatz (10) nach Anspruch 3,
**dadurch gekennzeichnet, dass**
das Zugelement (31)
- innerhalb, bevorzugt am Innendurchmesser, oder außerhalb, bevorzugt am Außendurchmesser, des Kraft- und Drehmomentübertragungsschlauches (3) angeordnet ist und/oder
- zumindest in einem proximalen und einem distalen Endbereich (311) des Kraft- und Drehmomentübertragungsschlauches (3) mit dem Kraft- und Drehmomentübertragungsschlauch (3) verbunden ist, bevorzugt verschweißt und/oder
- ein Seil, bevorzugt ein Metallseil, ein Draht, eine Faser oder ein Faserbündel ist.

5. Arbeitseinsatz (10) nach Anspruch 3 oder 4,
**dadurch gekennzeichnet, dass**
- die Arbeitsvorrichtung (2) über ein Gelenk mit dem Schaft (1) verbunden ist, über das die Arbeitsvorrichtung (2) um eine normal zur Längsachse des Schafts (1) verlaufende Drehachse abwinkelbar ist, und/oder
- der Schaft (1) in einem abwinkelbaren Schaftabschnitt (14) zumindest ein Gelenk (6) aufweist, bevorzugt zwei oder mehr Gelenke (6), über das/die dieser um eine normal zur Längsachse des Schafts (1) verlaufende Drehachse abwinkelbar ist und/oder in zumindest einem Längenabschnitt biegsam ist, wobei die Kraft- und Drehmomentübertragungsvorrichtung bevorzugt in dem abwinkelbaren Schaftabschnitt (14) als Kraft- und Drehmomentübertragungsschlauch (3) ausgebildet ist,
- der Schaft (1) längenverstellbar ist, bevorzugt teleskopierbar.

6. Arbeitseinsatz (10) nach zumindest einem der Ansprüche 3 bis 5,
**dadurch gekennzeichnet, dass**
der Arbeitseinsatz (10) ein elektrochirurgischer Arbeitseinsatz (10) ist, wobei sich entlang des Schafts (1) zumindest eine elektrische Verbindungsleitung (32) von dem Kopplungsabschnitt (102) zu dem distalen Werkzeugende (101) erstreckt, die mit zumindest einer der Branchen (21,22) elektrisch verbunden ist.

7. Arbeitseinsatz (10) nach Anspruch 6,
**dadurch gekennzeichnet, dass**
die zumindest eine elektrische Verbindungsleitung (32) im Inneren des Kraft- und Drehmomentübertragungsschlauchs (3) verläuft, bevorzugt an einer dem Zugelement (31) gegenüberliegenden Umfangsposition oder zentrisch.

8. Mikrochirurgisches Instrument (100),
das eine proximale Handhabe (12) mit zumindest einem Betätigungselement (4,5,7) und einen distalen Arbeitseinsatz aufweist, der mit seinem Kopplungsabschnitt operativ mit einer korrespondierenden Kopplungsvorrichtung der Handhabe (12) gekoppelt ist,
**dadurch gekennzeichnet, dass**
der Arbeitseinsatz ein Arbeitseinsatz (10) nach zumindest einem der Ansprüche 3 bis 7 ist.

9. Mikrochirurgisches Instrument (100) nach Anspruch 8,
**dadurch gekennzeichnet, dass**
die Handhabe (12)
- als Pistolengriff oder Pinzettengriff ausgebildet ist und/oder
- zumindest zwei mechanische Betätigungselemente (4,5,7) aufweist, ein erstes Betätigungselement (4) zur Ausübung eines Drehmoments auf die Kraft- und Drehmomentübertragungsvorrichtung des Arbeitseinsatzes (10) und ein zweites Betätigungselement (5) zur Ausübung einer Kraft in Längsrichtung der Kraft- und Drehmomentübertragungsvorrichtung des Arbeitseinsatzes (10).

10. Mikrochirurgisches Instrument (100) nach Anspruch 9,
**dadurch gekennzeichnet, dass**
zumindest eines der mechanischen Betätigungselemente (4,5,7)
- ein manuelles Betätigungselement (4,5) ist, bevorzugt ein Drehrad, ein Hebel, ein Druckknopf, und/oder
- ein Betätigungselement (7) ist, über das eine Antriebsvorrichtung aktivierbar ist, die dazu ausgebildet ist, auf die Kraft- und Drehmomentübertragungsvorrichtung des Arbeitseinsatzes (10) einzuwirken, wobei die Antriebsvorrichtung bevorzugt ein Dreh- und/oder Linearantrieb ist, besonders bevorzugt ein elektrischer oder pneumatischer Dreh- und/oder Linearantrieb.

## Claims

1. A power and torque transmission hose (3) for a work device (10) for a microsurgical instrument (100),
**characterized in that**
the power and torque transmission hose (3), at least in a portion situated between its two ends, is stiffened by at last one pulling element (31) running in the longitudinal direction of the power and torque transmission hose (3).

2. The power and torque transmission hose (3) according to claim 1, **characterized in that**
the pulling element (31)
- is positioned inside, preferably on the inner diameter, or outside, preferably on the outer diameter, of the power and torque transmission hose (3) and/or
- is connected, at least in a proximal and a distal (311) end portion of the power and torque transmission hose (3) with the power and torque transmission hose (3), preferably by welding, and/or
- is a rope, preferably a metallic rope, a wire, a fiber or a fiber bundle.

3. A work device (10) for a microsurgical instrument (100) having
- a proximal coupling portion (102), which can be coupled with a handle (12),
- wherein a hollow shaft (1) extends from the coupling portion (102) to a distal tool end (101) on which is positioned a jaw-shaped work apparatus (2), said work apparatus (2) having two arms (21,22) which can pivot with respect to one another, and said work apparatus (2) being mounted so that it can rotate about the longitudinal axis of the shaft (1),
- and wherein a power and torque transmission apparatus, inside the shaft (1), extends from the coupling portion (102) to the distal tool end (101), said apparatus being configured along one longitudinal portion as a power and torque transmission hose (3), so that the power and torque transmission apparatus is operatively connected with the work apparatus (2) to transmit a rotary motion and opening and closing forces,
**characterized in that**
the power and torque transmission hose is a power and torque transmission hose (3) according to claim 1 or 2 and is stiffened at least in one longitudinal portion with at least one pulling element (31) running in the longitudinal direction.

4. The work device (10) according to claim 3,
**characterized in that**
the pulling element (31)
- is positioned inside, preferably on the inner diameter, or outside, preferably on the outer diameter, of the power and torque transmission hose (3) and/or
- is connected, at least in one proximal and one distal end portion (311) of the power and torque transmission hose (3), with the power and torque transmission hose (3), preferably by welding, and/or
- is a rope, preferably a metallic rope, a wire, a fiber or fiber bundle.

5. The work device (10) according to claim 3 or 4,
**characterized in that**
- the work apparatus (2) is connected by a joint with the shaft (1), by which the work apparatus (2) can be turned about a rotary axis running perpendicular to the longitudinal axis of the shaft (1), and/or
- the shaft (1) comprises in a bendable shaft segment (14) at least one joint (6), preferably two or more joints (6), by which it can be turned about a rotary axis running perpendicular to the longitudinal axis of the shaft (1) and/or in at least one longitudinal portion is pliable, wherein the power and torque transmission apparatus, preferably in the bendable shaft segment (14), is configured as a power and torque transmission hose (3),
- the shaft (1) can be longitudinally adjusted, preferably telescoped.

6. The work device (10) according to at least one of claims 3 to 5,
**characterized in that**
the work device (10) is an electro-surgical work device (10), wherein at least one electrical connection line (32) extends along the shaft (1) from the coupling portion (102) to the distal tool end (101) and is electrically connected with at least one of the arms (21,22).

7. The work device (10) according to claim 6,
**characterized in that**
the at least one electrical connection line (32) runs in the interior of the power and
torque transmission hose (3), preferably at a peripheral position opposite the pulling element (31) or centered.

8. A microsurgical instrument (100),
which comprises a proximal handle (11) having at least one actuation element (4,5,7) and a distal work device, which is operatively coupled with a corresponding coupling apparatus of the handle (12) by its coupling portion,
**characterized in that**
the work device is a work device (10) according to at least one of claims 3 to 7.

9. The microsurgical instrument (100) according to claim 8,
**characterized in that**
the handle (12)
- is configured as a pistol grip or pincers grip and/or
- comprises at least two mechanical actuation elements (4,5,7), a first actuation element (4) to exert torque on the power and torque transmission apparatus of the work device (10) and a second actuation element (5) to exert a force in the longitudinal direction of the power and torque transmission apparatus of the work device (10).

10. The microsurgical instrument (100) according to claim 9,
**characterized in that**
at least one of the mechanical actuation elements (4,5,7)
- is a manual actuation element (4,5), preferably a rotary wheel, a lever, a pushbutton, and/or
- is an actuation element (7) by which a power drive apparatus can be actuated, said power drive apparatus being configured to act on the power and torque transmission apparatus of the work device (10), wherein the power drive apparatus is preferably a rotary and/or linear power drive, especially preferably an electrical or pneumatic rotary and/or linear power drive.

## Revendications

1. Flexible de transmission de la force et du couple (3) pour un embout de travail (10) pour un instrument microchirurgical (100),
**caractérisé en ce que**
le flexible de transmission de la force et du couple (3) est rigidifié au moins dans une section située entre ses deux extrémités, avec au moins un élément de traction (31) s'étendant dans le sens longitudinal du flexible de transmission de la force et du couple (3).

2. Flexible de transmission de la force et du couple (3) selon la revendication n°1,
**caractérisé en ce que**
l'élément de traction (31),
- est disposé à l'intérieur, de préférence sur le diamètre intérieur, ou à l'extérieur, de préférence sur le diamètre extérieur, du flexible de transmission de la force et du couple (3) et/ou
- est relié, de préférence soudé, au moins dans une zone d'extrémité proximale et une zone d'extrémité distale (311) du flexible de transmission de la force et du couple (3), au flexible de transmission de la force et du couple (3)
et/ou
- est un câble, de préférence un câble métallique, un fil métallique, une fibre ou un faisceau de fibres.

3. Embout de travail (10) pour un instrument microchirurgical (100),
- avec une section de couplage (102) proximale qui peut être couplée avec une poignée (12),
- une tige creuse (1) s'étendant de la section de couplage (102) vers une extrémité d'outil distale (101), sur laquelle est disposé un dispositif de travail (2) en forme de mâchoire, monté en rotation autour de l'axe longitudinal de la tige (1), avec au moins deux branches (21, 22) pivotantes l'une par rapport à l'autre,
- et un dispositif de transmission de la force et du couple qui est conçu, au moins le long d'une section de longueur, comme flexible de transmission de la force et du couple (3), s'étendant à l'intérieur de la tige (1), depuis la section de couplage (102) vers l'extrémité d'outil distale (101), le dispositif de transmission de la force et du couple pour la transmission d'un mouvement de rotation et de forces d'ouverture et de fermeture étant opérationnellement relié au dispositif de travail (2),
**caractérisé en ce que**
le flexible de transmission de la force et du couple est un flexible de transmission de la force et du couple (3) selon la revendication n°1 ou n°2, qui est rigidifié, au moins dans une section de longueur, avec au moins un élément de traction (31) s'étendant dans le sens longitudinal.

4. Embout de travail (10) selon la revendication n°3,
**caractérisé en ce que**
l'élément de traction (31)
- est disposé à l'intérieur, de préférence sur le diamètre intérieur, ou à l'extérieur, de préférence sur le diamètre extérieur, du flexible de transmission de la force et du couple (3) et/ou
- est relié, de préférence soudé, au moins dans une zone d'extrémité proximale et une zone d'extrémité distale (311) du flexible de transmission de la force et du couple (3), au flexible de transmission de la force et du couple (3)
et/ou
- est un câble, de préférence un câble métallique, un fil métallique, une fibre ou un faisceau de fibres.

5. Embout de travail (10) selon la revendication n°3 ou n°4,
**caractérisé en ce que**
- le dispositif de travail (2) est relié à la tige (1) par une articulation, par laquelle le dispositif de travail (2) peut être coudé autour d'un axe de rotation s'étendant normalement par rapport à l'axe longitudinal de la tige (1) et/ou
- la tige (1) présente, dans une section de tige (14) pouvant être coudée, au moins une articulation (6), de préférence deux ou plusieurs articulations (6), par laquelle/lesquelles celui-ci peut être coudé autour d'un axe de rotation s'étendant normalement par rapport à l'axe longitudinal de la tige (1) et/ou est flexible au moins dans une section de longueur, le dispositif de transmission de la force et du couple étant conçu comme flexible de transmission de la force et du couple (3), de préférence dans la section de tige (14) pouvant être coudée,
- la tige (1) est réglable en longueur, de préférence télescopable.

6. Embout de travail (10) selon au moins une des revendications n°3 à n°5,
**caractérisé en ce que**
l'embout de travail (10) est un embout de travail électro-chirurgical (10), au moins une ligne de raccordement électrique (32) s'étendant le long de la tige (1), depuis la section de couplage (102) vers l'extrémité d'outil distale (101) qui est reliée électriquement au moins à l'une des branches (21, 22).

7. Embout de travail (10) selon la revendication n°6,
**caractérisé en ce que**
l'au moins une ligne de raccordement électrique (32) passe à l'intérieur du flexible de transmission de la force et du couple (3), de préférence sur une position périphérique opposée à l'élément de traction (31) ou de manière centrée.

8. Instrument microchirurgical (100),
présentant une poignée (12) proximale avec au moins un élément d'actionnement (4, 5, 7) et un embout de travail distal qui est, avec sa section de couplage, opérationnellement couplé avec un dispositif de couplage correspondant de la poignée (12),
**caractérisé en ce que**
l'embout de travail est un embout de travail (10) selon au moins une des revendications n°3 à n°7.

9. Instrument microchirurgical (100) selon la revendication n°8,
**caractérisé en ce que**
la poignée (12)
- est conçue comme poignée pistolet ou poignée pincette et/ou
- présente au moins deux éléments d'actionnement (4, 5, 7) mécaniques, un premier élément d'actionnement (4) pour exercer un couple sur le dispositif de transmission de la force et du couple de l'embout de travail (10) et un deuxième élément d'actionnement (5) pour exercer une force dans le sens longitudinal du dispositif de transmission de la force et du couple de l'embout de travail (10).

10. Instrument microchirurgical (100) selon la revendication n°9,
**caractérisé en ce que**
au moins l'un des éléments d'actionnement (4, 5, 7) mécaniques
- est un élément d'actionnement (4, 5) manuel, de préférence une molette, un levier, un bouton-poussoir et/ou
- est un élément d'actionnement (7) permettant d'activer un dispositif d'entraînement qui est conçu pour agir sur le dispositif de transmission de la force et du couple de l'embout de travail (10), le dispositif d'entraînement étant de préférence un entraînement rotatif et/ou linéaire, en particulier de préférence un entraînement rotatif et/ou linéaire électrique ou pneumatique.
